Europäisches Patentamt

European Patent Office

Office européen des brevets

(11) Publication number: **0 411 896 A2**

# EUROPEAN PATENT APPLICATION

(21) Application number: 90308407.7

(22) Date of filing: 31.07.90

(51) Int. Cl.5: **C12N 1/38**, C12N 15/36, C12N 15/80

(30) Priority: 31.07.89 US 387680

(43) Date of publication of application:
06.02.91 Bulletin 91/06

(84) Designated Contracting States:
BE CH DE FR GB IT LI NL

(71) Applicant: MERCK & CO. INC.NC.
126, East Lincoln Avenue P.O. Box 2000
Rahway New Jersey 07065-0900(US)

(72) Inventor: Schulman, Cheryl A.
84 Pine Valley Road
Doylestown, PA 18901(US)
Inventor: Kniskern, Peter J.
841 Patterson Drive
Lansdale, PA 19446(US)
Inventor: Maigetter, Robert Z.
4840 Mechanicsville Road
Mechanicsville, PA 18934(US)

(74) Representative: Hesketh, Alan, Dr. et al
European Patent Department Merck & Co.,
Inc. Terlings Park Eastwick Roadoad
Harlow Essex, CM20 2QR(GB)

(54) Growth medium and fermentation process.

(57) A fermentation process as well as a medium for producing recombinant proteins in a host strain containing the desired mutation in cell wall and/or plasma membrane biosynthesis (e.g. mnn9 ) is disclosed. An example of such a strain is described in Merck's pending application Serial No. 202,764 filed June 3, 1988, corresponding to EP-A-0344864.

EP 0 411 896 A2

## GROWTH MEDIUM AND FERMENTATION PROCESS

### BACKGROUND OF THE INVENTION

The description of the genotype of the mnn9 gene of S. cerevisiae was disclosed by Ballou et. al., (1980 Saccharomyces cerevisiae mutants that make mannoproteins with a truncated carbohydrate outer chain. J. Biol. Chem. 255 :5986-5991) and Tsai et. al, (1984 Carbohydrate structure of Saccharomyces cerevisiae mnn9 mannoprotein. J. Biol. Chem. ·260:3805-3811). Strains of yeast with the mnn9 genotype are unable to N-glycosylate proteins beyond the core mannan backbone (please see Gopal and Ballou. PNAS 84 :8824 (1987). Cells containing the mnn9 mutation are grown typically in YEPD medium (1% yeast extract/2% Bacto-peptone/2% D-glucose) as described in J. Biol. Chem. 255 :5986-5991; the cells generated from these fermentations have been used to study the biochemistry of yeast glycosylation.

One consequence of the mnn9 mutation on the yeast is an increased fragility of the cell as described in Ballou (1980. J. Biol. Chem. 255 :5986-5991). It has been suggested that this fragility can be compensated for by adding sorbitol to the medium. Occasionally, revertant MNN9 strains would overgrow the mnn9 cells in YEPD medium.

It is the object of the present invention to compensate for this fragility through adjustments to medium composition and growth parameters to thereby prevent overgrowth of the mnn9 cultures by MNN9 revertants with the consequent changes in the recombinant product.

It is obvious to those skilled in the art that the invention described herein extends to other hosts and mutations in host cell wall and/or membrane biosynthesis other than mnn9 .

### SUMMARY OF THE INVENTION

The present invention relates to a medium as well as a method for optimizing the osmotic stability of this medium. Such a medium is designed to maximize the productivity of heterologous proteins in cells harboring mutations in cell wall and/or plasma membrane biosynthesis, for example, the mnn9 mutation. More specifically, the present invention relates to a medium with an assimilable source of nitrogen, amino acids, and an assimilable carbon source whose osmolarity is adjusted either through increased concentrations of medium components or by the addition of an extraneous osmotic stabilizer.

As used hereinafter and in the claims, the following terms have their meaning defined:

Osmotic Stabilizer: any compound that, when added to the growth medium, increases the osmolarity of the medium. These include, for example, electrolytes such as NaCl, sugars such as sorbitol, polyhydric alcohols such as glycerine etc.

mnn9 Yeast: strains of S. cerevisiae containing the mnn9 genotype, which are described in Merck's pending U.S. Patent Application No. 202,764, corresponding to EP-A-0344864, and incorporated herein by reference.

Foreign DNA: fragment(s) of non-host DNA that encode a heterologous proteins or glycoprotein(s).

Heterologous Protein Production or Expression: production of a non-yeast protein in yeast cells. The DNA required to produce this protein can be contained within one of the chromosomes of the yeast genome (intergrated) or may be present extrachromosomally on a suitable plasmid vector.

Plasmid Vector: an extrachromosomal segment of DNA capable of autonomous replication in yeast that is able to incorporate other fragments of DNA that encode and/or direct the synthesis and expression of a heterologous protein.

Genome: the DNA within a cell that defines the characteristics of that cell.

Milliosmolarity (mOsm): the strength of the tendency of water to flow from a solution containing a higher concentration of solutes to a solution containing a lower concentration of solutes. The solution of solutes can be separated from one another by any semi-permeable membrane, including, but not limited to, the membrane surrounding living cells. Yeast possess such a semi-permeable membrane.

Broadly speaking, the method according to this invention comprises:

1) growing under suitable nutrient conditions S. cerevisiae cells harboring the desired mutation (e.g. mnn9 mutation) in their genome and also containing a suitable fragment of DNA for the expression of a heterologous protein in yeast. This fragment could be either integrated into the yeast genome or present on a DNA plasmid vector.

2) controlling the environmental conditions within the nutrient medium to maximize production of the heterologous protein and simplify isolation of the heterologous protein, while maintaining optimal cellular growth of the mutant cells.

The present invention also encompasses within its scope media for the production of heterologous proteins using strains of yeast containing other mutations that affect the ability of cells to grow and produce heterologous proteins in the absence of osmotic stabilization (among others, mutants in structural compounds of the yeast cell wall and/or plasmid membrane).

Other aspects and advantages of the present invention will become apparent to those skilled in the art upon consideration of the following detailed description which provides illustrations of the practice of the invention in its presently preferred embodiments without, however, limiting the same hereto.

## DETAILED DESCRIPTION OF THE INVENTION

In a typical practice according to this invention, a strain of S. cerevisiae harboring the mutant genotype (e.g. mnn9 ) and a suitable fragment of foreign DNA plasmid vector is used to produce various heterologous proteins. Osmotic stabilization of the medium is required to minimize any growth advantage that wild-type MNN9 yeast cells may have over mutant mnn9 yeast cells. For yeast cells containing the mnn9 mutation, optimal production of heterologous proteins and maximal cellular growth occur when the osmolarity of the medium in which the cells are present is between 533 and 1050 mOsm, typically 533 to 790 mOsm.

It is obvious to those skilled in the art that the optimal range of osmolarity may be different for other host cells harboring mutations other than mnn9 which induce cellular fragility.

The osmolarity of the medium is determined in any suitable fashion. For example, an Osmette A, a device manufactured by Precision Systems, Inc. of Sudbury, Massachusettes might be used. The osmolarity of the sample, relative to standards of known osmolarity is calculated based on a measurement of freezing-point depression.

In order to establish a model to evaluate media and process parameters, the middle protein of the Hepatitis B virus envelope (also called preS2 + S) was chosen as a model heterologous protein. As described in detail elsewhere (please see Kniskern et al 1989, Ellis et al 1988) the choice of preS2 + S protein is biomedically relevant as this protein assembles into a form in the recombinant host cells which confers on it the properties of an effective immunogen as well as, potentially, an assay reagent in that it displays important protective epitopes of the hepatitis B virus. It is however obvious to those skilled in the art that any foreign reporter gene could have been chosen and as such could include the other envelope genes of the hepatitis B virus (S and preS1 + preS2 + S) which also have similar biomedical relevance.

The production of this heterologous reporter protein (preS2 + S) is measured in crude cell lysates by radioimmunoassay (RIA) and immunobolt analysis using antibody specific for the particular protein product according to the methods described by Ellis et al. (1988. Preparation and testing of a recombinant-derived hepatitis B vaccine consisting of preS2 + S polypeptides. in : Viral Hepatitis and Liver Disease, A.J. Zuckerman (ed.), Alan R. Liss, New York, pp 1079-1086). Cellular mass production is determined using either light scatter at a wavelength of 660 nm (absorbance, A660) or by dry cell weight determinations.

It is obvious to those skilled in the art that the selection of host strains of S. cerevisiae extends to all strains containing mutation which result in increased fragility due to changes in the osmolarity of the medium. It is also obvious to those skilled in the art that expression in such mutants extends to species and cells derived from, but not limited to mammals, insects, bacteria, and other fungi.

The genus Saccharomyces is composed of a variety of species. S. cerevisiae is commonly used as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess promoters which are analogous or identical to promoters in S. cerevisiae . Therefore, it will be obvious to those skilled in the art that, for the expression of preS2 + S polypeptides, the selection of a host strain extends to other species of the genus Saccharomyces , including, but not limited, to carlsbergensis , diastaticus , elongisporus , kluyveri , montanus , norbensis , oviformis , rouxii , and uvarum .

Several yeast genera such as Candida , Hansenula , Pichia , and Torulopsis have been shown to contain similar metabolic pathways for the utilization of methanol as a sole carbon sourcee for growth. The gene for alcohol oxidase, an enymze which participates in this metabolic pathway, has been isolated from Pichia pastoris . The P. pastoris alcohol oxidase promoter has been isolated and shown to be susceptible to methanol induction of expression. Such an inducible promoter is useful for the expression of polypeptides in yeast. In particular, this promoter has been shown to be active son a plasmid for the inducible expression of the S domain in P. pastoris in particulate form. This observation highlights the ability of other yeast genera to function as hosts for the recombinant DNA-mediated expression of polypeptides in immunologically-active form. Therefore, it will be obvious to those skilled in the art that, for the expression of preS2 + S, the selection of a host strain extends to species from other genera of yeast from the Families Saccharomycetaceae and Cryptococcaceae, including, but not limited to Candida , Hansenula , Kluyveromyces , Pichia , Saccharomyceopsis , and Torulopsis .

The following examples serve to further illustrate the embodiments of the present invention without, however, limiting the same thereto.

EXAMPLE 1

Production of Hepatitis B Surface Antigen PreS2 + S

Production of preS2 + S was directed from a DNA plasmid vector containing the galactose-regulated GAL10 promoter, PreS2 + S ORF, and ADH1 terminator (expression cassette) as described in Kniskern et al [(1989) Proceedings of the Fifth International Symposium on Immunobiology of Proteins and Peptides].

Cells of S. cerevisiae containing the mnn9 mutation and the DNA plasmid vector with the expression cassette were grown in 250-mL shake flasks containing 50-mL of the media listed in Table 1.

TABLE 1

| Composition of Media Used in Example 1 | | | | | |
|---|---|---|---|---|---|
| Medium # | Yeast Extract g/L | Soy Peptone g/L | Glucose g/l | Sorbitol M | Osmolarity mOsm |
| A | 40 | 20 | 40 | 0.0 | 533 |
| B | 40 | 20 | 40 | 0.1 | 655 |
| C | 40 | 20 | 40 | 0.25 | 790 |
| D | 40 | 20 | 40 | 0.5 | 1050 |
| E | 40 | 20 | 40 | 1.0 | 1572 |
| F | 40 | 20 | 40 | 1.2 | 1782 |

Following inoculation, flasks were aerated by agitation in a 28°C constant temperature incubator. When the concentration of glucose in the media was less than 10 g/L, galactose was added to the medium to a final concentration of 1-4% (w/v); the galactose induces the production of preS2 + S when the glucose in the medium has been depleted. Incubation continued as described above for 2 more days. Cellular mass was determined spectrophotometrically. Pellets of cells were prepared and subsequently analyzed for preS2 + S production by breaking the cells with glass beads and assaying by RIA and Lowry protein (please see Ellis et al 1988, Preparation and testing of a recombinant derived hepatitis B vaccine consisting of preS2 + S polypeptides. Viral Hepatitis and Liver Disease (A.J. Zuckerman, ed.). Alan R. Liss, NY. pp 1079-1086.

The data are presented in Table 2.

TABLE 2

| Effect of Sorbitol on Cellular Growth and HBsAg PreS2 + S Expression | | | |
|---|---|---|---|
| Medium | Sorbitol Added (M) | Cellular Growth (A660) | PreS2 + S Expression Relative Sp. Act (Units/mg protein) |
| A | 0.0 | 18.8 | 4.3 |
| B | 0.1 | 22.0 | 3.4 |
| C | 0.25 | 20.8 | 2.1 |
| D | 0.5 | 18.4 | 1.0 |
| E | 1.0 | 12.4 | 1.7 |
| F | 1.2 | 8.6 | 0.7 |

As the concentration of sorbitol in the medium increased above 0.1M, the cellular mass decreased and the amount of HBsAg PreS2 + S per unit protein (specific activity) decreased.

EXAMPLE 2

Production of HBsAg preS2 + S in Fermentors

A vial containing the frozen recombinant yeast was thawed and the contents were inoculated onto a plate of seed medium (5XLEU/HSA: 0.85% yeast nitrogen base without amino acids and ammonium sulfate/0.02% adenine/0.025% tyrosine/0.02% uracil/21.8% sorbitol/0.5% ammonium sulfate/4% glucose/0.0032% zinc sulfate/0.0038% ferric chloride/0.01% L-arginine/0.005% L-histidine-HCl/0.03% L-isoleucine/0.02% L-lysine-HCl/0.005% L-methionine/0.03% L-phenylalanine/0.02% L-tryptophan/2% agar). The plate was incubated at 28° C for 3 days. The growth from the surface of the plate was inoculated into a 2-L flask containing 500 mL of seed medium (YEHD/HS: 2% yeast extract/1% soy peptone/2% glucose/21.8% sorbitol). The flask was incubated at 28° C and 350 RPM for 20 hours. The contents of the flask were then inoculated into a 16-L fermentor containing 11 L of YEHD/HS. The fermentor was operated at 28° C, 500 RPM, and 5 L/min. air. After 11 hours incubation, the contents of the 16-L fermentor were transferred into a 250-L fermentor containing 200-L of production medium (2XYEHD/HS: 4% yeast extract/2% soy peptone/4% glucose/21.8% sorbitol). The fermentor was operated at 28° C, 260 RPM, and 90 L/min air. After 16 hours incubation, 10 L of a 40% galactose solution was added to the fermentor. The fermentation was concluded after 57 hours of incubation and the yeast cells were harvested.

Alternatively, a vial containing frozen yeast stock was thawed and the contents inoculated into a 250-mL flask containing 50 mL of seed medium (5XLEU/LS: 0.85% yeast nitrogen base without amino acids and ammonium sulfate/0.02% adenine/0.025% tyrosine/0.02% uracil/1.0% succinic acid/0.79% (v/v) 50% NaOH/1.82% sorbitol/0.5% ammonium sulfate/4% glucose/0.0032% zinc sulfate/0.0038% ferric chloride/0.01% L-arginine 0.005% L-histidine-HCl/0.03% L-isoleucine/0.02% L-lysine-HCl/0.005% L-methoinine/0.03% L-phenylalanine 0.02% L-tryptophan).

The flask was incubated at 28° C and 350 RPM for 19 hours. A 2-L flask containing 500 mL of 5XLEU/LS was inoculated with 25 mL from the first flask and incubated as described above for 12 hours. The contents of the 2-L flask were inoculated into a 16-L fermentor containing 10 L of 5XLEU/LS. The fermentor was operated at 28° C, 500 RPM, and 5 L/min air for 12 hours. The contents of the 16-L fermentor were then inoculated into a 250-L fermentor containing 200 L of 2XYEHD/LS (4% yeast extract/2% soy peptone/4% dextrose/1.8% sorbitol). The 250-L fermentor was operated 28° C, 260 RPM, and 60 L/min air. After 14 hours incubation, 10 L of a 40% galactose solution was added to the fermentor to induce production of HBsAg preS2 + S. The fermentation was ended after 59 hours of growth and the yeast alls were harvested.

Fermentor and shake flask studies comparing the two methods of preparing the seed cultures for

5

inoculation into the 250-L fermentor have indicated that the methods yield no differences in productivity.

Samples were taken at the end of each fermentation for growth and (preS2 + S) productivity and the results are summarized in Table 3. The fermentor grown in the medium containing lower sorbitol (2XYEHD/LS) yielded a higher cellular mass and higher antigen:protein ratio than did the fermentor grown in the medium containing the higher concentration of sorbitol (2XYEHD/HS).

TABLE 3

| Effect of Sorbitol Concentration on Cellular Growth and Production of preS2 + S in 250-L Fermentors | | | |
|---|---|---|---|
| Medium | Sample Age (h) | Cellular Growth (DCW: g/L) | Antigen:Protein Ratio (mg/mg) |
| 2XYEHD/HS | 57 | 9 | 5.4 |
| 2XYEHD/LS | 59 | 27 | 13.3 |

EXAMPLE 3

Production of preS2 + S in Fermentors

A frozen culture of recombinant yeast was thawed and the contents inoculated onto 5XLEU/HSA. The plate was incubated at 28°C for 3 days. Growth from the surface of the plate was inoculated into 2-L seed flasks containing 500 mL of media. The flasks were incubated at 28°C and 350 RPM. After 14-20 hours incubation, 75 mL from the seed flasks were inoculated into 2-L fermentors containing 1.5 L of media. The same media were used for the seed flasks as were used in the fermentors. Formulations for the media used are listed in Table 4.

TABLE 4

| Media Used in Example 3 | | | | | |
|---|---|---|---|---|---|
| | Medium Components* | | | | |
| Medium/Sorbitol Concentration | Yeast Extract | Soy Peptone | Dextrose | Sorbitol | Osmolarity Mosm |
| 2XYEHD/OMS | 4 | 2 | 4 | 0 | 533 |
| 2XYEHD/0.1MS | 4 | 2 | 4 | 1.82 | 655 |
| 2XYEHD/1MS | 4 | 2 | 4 | 18.2 | 1572 |
| 2XYEHD/1.2MS | 4 | 2 | 4 | 21.8 | 1782 |

* Concentrations listed as % (w/v).

The concentration of glucose in the media was monitored; when the concentration of glucose dropped below 10 g/L, a solution of galactose was added to each tank to a final concentration of 2% (w/v) to induce expression of preS2 + S. Samples were removed from the fermentors at the conclusion of the experiment and were assayed for cellular growth and preS2 + S antigen:protein ratios. These data are presented in Table 5.

TABLE 5

| Comparison of Cellular Yields and preS2 + S Productivity in 2XYEHD with 0M, 0.1M, 1.0M, and 1.2M Sorbitol in Fermertors | | | | |
|---|---|---|---|---|
| | Concentration of Sorbitol in Medium | | | |
| | 0M | 0.1M | 1.0M | 1.2M |
| Cellular Yield (g/L DCW) | 11.4 | 17.5 | 13.3 | 10.8 |
| Specific Activity (mg preS2 + S/mg protein) | 16.2 | 10.5 | 4.1 | 3.7 |
| Volumetric Productivity (mg/L) | 54.6 | 54.2 | 7.2 | 19.0 |

Cellular yield in the fermentors increased as the sorbitol concentration (and osmolarity) decreased. Likewise, the antigen:protein ratio increased with decreasing osmolarity.

**Claims**

1. A medium for the growth of recombinant cells expressing foreign proteins comprising an assimilable source of nitrogen, amino acids, sugar, and trace elements in which the molar concentrations of medium components are configured yielding an osmolarity optimal for host cell growth .

2. A medium for the growth of recombinant cells expressing foreign proteins containing sources of nitrogen, amino acids, an energy source, and an osmotic stabilizer at concentrations adjusted to obtain an osmolarity optimal for host cell growth.

3. A medium according to Claims 1 & 2 wherein the osmolarity is between 533 mOsm and 1050 mOsm.

4. A medium according to Claim 3 comprising yeast extract, soy peptone, and dextrose.

5. A medium according to Claim 4 wherein the concentrations of the medium components are: yeast extract = 40-50 g/L, soy peptone = 20-30 g/L, dextrose = 40-50 g/L, and the osmolarity is between 533 mOsm and 1050 mOsm.

6. A medium according to Claim 1 & 2 wherein the recombinant host cells are a yeast or fungus.

7. A medium according to Claim 6 wherein the host cell is a species of yeast derived from the Families Saccharomycetaceae or Cryptococcaceae .

8. A medium according to Claim 7 wherein the yeast species is from the genus Saccharomyces .

9. A medium according to Claim 8 wherein the yeast species is Saccharomyces cerevisiae .

10. A process for the production of heterologous proteins comprising cultivation of S. cerevisiae with a mutation or mutations in the components for cell wall or plasma membrane biosynthesis and an expression cassette suitable for production of the heterologous protein in a medium as defined in Claim 1.

11. A process according to Claim 10 wherein the mutation is in a gene for glycoprotein biosynthesis.

12. A process according to Claim 11 wherein the mutation is in the mnn9 gene.

13. A process for the production of heterologous proteins consisting of inducing production of a heterologous protein in mnn9 yeast cells with a mutation or mutations in the biosyntheses of cell walls or plasma membrane component(s) containing an expression cassette suitable for the production of a heterologous protein in a medium according to Claim 1.

14. A process according to Claim 13 wherein the mutation is in a gene for glycoprotein biosynthesis.

15 . A process according to Claim 13 wherein the mutation is in the mnn9 gene.

16. A process for the production of HBsAg preS2 + 2 comprising cultivation of cells of S. cerevisiae with the mnn9 genotype and a yeast DNA plasmid containing an expression cassette for as hepatitis B virus envelope protein A process according to Claim 14 wherein the hepatitis B virus envelope protein is preS2 + S in a medium according to Claim 1.

17. A process according to Claim 16 wherein the hepatitis B virus envelope protein is preS2 + S.